# EUROPEAN PATENT APPLICATION

(11) **EP 1 214 909 A1**
(43) Date of publication of application: **19.06.2002**
(21) Application number: 00960985.0
(22) Date of filing: 13.09.2000
(51) Int. Cl.: A61B 8/00

(54) **ULTRASONIC PROBE AND ULTRASONIC DIAGNOSTIC DEVICE COMPRISING THE SAME**

(30) Priority: 17.09.1999 JP 26303399; 14.12.1999 JP 35460199
(71) Applicant: Hitachi Medical Corporation, Tokyo 101-0047 (JP)
(72) Inventor: SANO, Shuzo, Kashiwa-shi, Chiba 277-0812 (JP); SATO, Yutaka, Kashiwa-shi, Chiba 277-0835 (JP); KUBOTA, Jun, Nagareyama-shi, Chiba 270-0176 (JP); KISHIMOTO, Shinji, Mitsukaido-shi, Ibaraki 303-0042 (JP); SHINOMURA, Ryuichi, Higashimatsuyama-shi, Saitama 355-0004 (JP); TAMANO, Satoshi, Kashiwa-shi, Chiba 277-0054 (JP); OSAWA, Takaya, Kitakatsushika-gun, Saitama 345-0025 (JP); MIWA, Yuichi c/o Central Res. Lab., Hitachi, Ltd., Kokubunji-shi, Tokyo 185-8601 (JP); MASUZAWA, H., Central Res. Lab., Hitachi Ltd., Kokubunji-shi, Tokyo 185-8601 (JP); UMEMURA, S., Central Res. Lab., Hitachi Ltd., Kokubunji-shi, Tokyo 185-8601 (JP); AZUMA, T., Central Res. Lab., Hitachi Ltd., okubunji-shi, Tokyo 185-8601 (JP)
(74) Representative: Strehl Schübel-Hopf & Partner
(86) International application number: PCT/JP00/06266
(87) International publication number: WO 01/21072

(57) **Abstract**

An ultrasonic probe in which two-dimensional array transducer elements are arrayed convexly both in one direction of the two-dimensional array and in the direction perpendicular to the former direction relative to the direction where an ultrasonic wave is transmitted. By switching a transducer element selecting switch circuit provided near the transducer elements, the shape of the diameter of the probe for transmitting and receiving of ultrasound and the position of the diameter are arbitrarily determined. An ultrasonic diagnostic apparatus in which a fresnel ring is formed as an diameter by a control signal to a transducer element selecting switch circuit, the number of cables for connection between the probe and the main body is decreased by bundling the transducer elements in one ring and effecting the connection, the fresnel ring is moved every ultrasonic transmission/reception cycle by the control signal to the transducer element selection switching circuit, and an object is three-dimensionally scanned with an ultrasonic beam.

## Description

### FIELD OF THE INVENTION

This invention relates to an apparatus for scanning an object to be examined three-dimensionally with an ultrasonic beam, particularly to an ultrasonic two-dimensional array probe being capable of scanning the object three-dimensionally with an ultrasonic beam by an electronic control and an ultrasonic diagnostic apparatus comprising it.

### BACKGROUND OF THE ART

Recently an ultrasonic diagnostic apparatus was developed that transmits and receives an ultrasonic beam with an ultrasonic probe, scans the object three-dimensionally, corrects three-dimensional data from the object, and makes a three-dimensional image for diagnosis. In the first example of such an apparatus, the three-dimensional image data is corrected by moving an ultrasonic probe with a plurality of transducer elements arranged in one direction parallel and mechanically on the surface of an object. The scanning is performed from side to side by inclining consecutively a contact angle of the probe to the object without changing the contact position between the probe and the object.

In addition, in a second example, a 2D-array ultrasonic probe is composed with 64×64 elements arrayed two-dimensionally. An ultrasonic beam with gimlet shape is transmitted and received in or from the interior of the object by using specified transducers fixedly selected in the 2D arrangement, and a three-dimensional image data from the object to be examined is corrected. In addition, the two-dimensional probe is a probe whose arrangement of elements is expanded from one direction to two directions. For example it is disclosed in Ultrasonic Imaging 14, 213-233 (1992); IEEE Trans. UFFC 38, 100-108 (1991).

However, the disadvantage of the traditional ultrasonic diagnostic apparatus is that the ultrasonic probe and the ultrasonic diagnostic apparatus have to be large and heavy because a driving system for the scanning ultrasonic beam and for moving the probe mechanically is necessary. Therefore, from an operational view, a small and light two-dimensional probe is desired. As the scanning range is basically restricted by the composition of the driving system, an arbitrary range cannot be scanned. Furthermore, the mechanical scanning mechanism causes abrasion. Therefore, the lifetime of the probe is short.

The disadvantage of the second ultrasonic apparatus is further that only the fixedly selected transducer elements in the two-dimensional array are used for transmitting and receiving an ultrasonic beam with gimlet shape. Therefore, the region for correcting the three-dimensional data is narrow. In addition, an even transducer element number used for transmitting and receiving is specified as a part of 64×64, but necessarily of about 256. So a lot of cables connected to each element are needed. If the number of transducer elements for transmitting and receiving ultrasound is increasing, a lot of beam forming circuits disposed on the main body of the diagnostic apparatus are also needed.

Thus in view of the previously described subject matter, the object of the present invention is to provide a small and light ultrasonic probe that is able to scan an object three-dimensionally by transmitting and receiving an ultrasonic beam but having a reduced number of cables for connecting the ultrasonic probe and the main body of the diagnostic apparatus and which two-dimensional array probe focus dynamically.

In another embodiment of the present invention a two-dimensional array probe is provided with is designed to easily contact the surface of the object to be examined.

A further embodiment of the present invention provides an ultrasonic probe that is able to scan to the object to be examined three-dimensionally with an ultrasonic beam without using a mechanical scanning mechanism.

In another embodiment of the present invention, an ultrasonic probe is provided with which a three-dimensional image data can be acquired from a wide range of objects to be examined.

A further embodiment of the present invention provides an ultrasonic diagnostic apparatus designed that an operator is not influenced by the weight and the hardness of cables during operation.

Furthermore, in another embodiment of the present invention the ultrasonic diagnostic apparatus is capable to transmit and receive an ultrasound with a two-dimensional array probe and has few beam forming circuits.

A further embodiment of the present invention provides an ultrasonic diagnostic apparatus, which obtains good quality images.

### DISCLOSURE OF THE INVENTION

In order to achieve the object, a first embodiment of the present invention comprises a plurality of transducer elements for transmitting and receiving an ultrasound, which are arrayed two-dimensionally and correct an ultrasonic signal. The plurality of transducer element is arrayed in one or two directions with convex shape to the transmitting direction of the ultrasound, wherein the two directions are perpendicular to each other. In case the ultrasonic probe has a convex shape in both directions, the transducer elements arrangement is preferable a radial arrangement in both directions, or a radial arrangement in one direction and a parallel arrangement in the other direction.

The ultrasonic probe of a further embodiment of the present invention has an element selecting switch circuit for selectively switching arbitrary transducer elements for transmitting and receiving ultrasound. The switch circuit is arranged in the vicinity of the two-dimensional array transducer elements. Output lines of the element selecting switch circuit are connected to each arrayed transducer element but the number of input lines is less than the number of the arrayed transducer elements.

In another embodiment of the present invention, an arbitrary transducer element can be selected by a control signal transmitted to the element selecting switch circuit. Accordingly, a diameter with an arbitrary shape can be formed. In addition, the diameter can be moved by transmitting the control signal to the element selecting switch circuit. With the motion of the diameter, a three-dimensional scan of the interior of an object is possible.

In another embodiment of the present invention, an ultrasonic diagnostic apparatus comprises the ultrasonic probe with two-dimensionally arrayed minute transducer elements. The apparatus further includes an element selecting means for supplying data to select transducer elements transmitting and receiving ultrasound from the array transducer of the ultrasonic probe, a means for supplying bundling data for bundling and connecting the selected transducer elements to a plurality of groups, a means for transmitting ultrasound to the object by applying a predetermined transmitting delay time to the bundled transducer element groups, a means for beam forming by receiving signals of the each bundled transducer groups, a means for image processing an output signal of this beam forming means, and an image display means.

In this embodiment the transducer elements are bundled to a plurality of groups and form a fresnel ring having a concentric circle. The ring is designed that the difference between a maximum and a minimum distance between the transducer elements in each ring forming the fresnel ring and the ultrasonic focus point, is less than 1/8 wavelength of the ultrasound.

In another embodiment of the ultrasonic diagnostic apparatus, the form of the fresnel ring is not changed during receiving an echo signal. Further, a means for controlling the beam forming circuit corresponding to the received signal of each ring is comprised for moving the receiving focus point continuously on the center axis line of the fresnel ring. Furthermore, the embodiment of the apparatus comprises a means for changing the form of the fresnel ring corresponding to the depth of the receiving focus point, a means for ultrasonic scanning the predetermined depth region of the object with each form of the fresnel ring, and a means for composing an image from an echo signal acquired at each depth region.

Moreover, an other embodiment of the ultrasonic diagnostic apparatus preferably comprises a two-dimensional array probe for transmitting and receiving ultrasound, a main body of the diagnostic apparatus for acquiring and displaying an ultrasonic image for the diagnosis of a part in the object's interior by using an ultrasonic signal corrected with the two-dimensional array probe, and a data transmitting part for transmitting selecting data for transducer elements and the corrected ultrasonic signal from one direction to another between the two-dimensional array ultrasonic probe and the main body.

### BRIEF DESCRIPTION OF THE DRAWING

Fig.1 is a perspective view showing an embodiment of an ultrasonic probe of the present invention for showing the interior structure of it.

Fig.2 is a perspective view showing the structure of one line of the transducer elements in the longitudinal direction.

Fig.3 shows a plurality of the transducer elements of Fig. 1 arranged in longitudinal direction.

Fig.4 shows a plurality of the transducer elements of Fig. 1 arranged in transversal direction.

Fig.5 is a view showing the outline of the element selecting switch circuit combined in the two- dimensional probe of Fig.1.

Fig.6 is a perspective view showing the interior structure of a modified example of the two-dimensional ultrasonic probe of the present invention.

Fig.7 is a block diagram showing an embodiment of an ultrasonic diagnostic apparatus of the present invention.

Fig.8 is a view showing a use embodiment of ultrasonic diagnostic apparatus shown in Fig.7.

Fig.9 is an operation diagram for scanning the object three-dimensionally with an ultrasonic beam with selected diameter.

Fig.10 is a view showing a fresnel bundled connection of the probe and a delay circuit.

Fig.11 is a view showing an ultrasound transmitted by fresnel bundling.

Fig.12 is a view showing an ultrasound received by fresnel bundling.

Fig.13 is a beam simulation view for the effectiveness of the fresnel bundling in the present invention.

### THE BEST MODE FOR CARRYING OUT THE INVENTION

Hereinafter embodiments of the present invention will be described in detail referring to the attached figure.

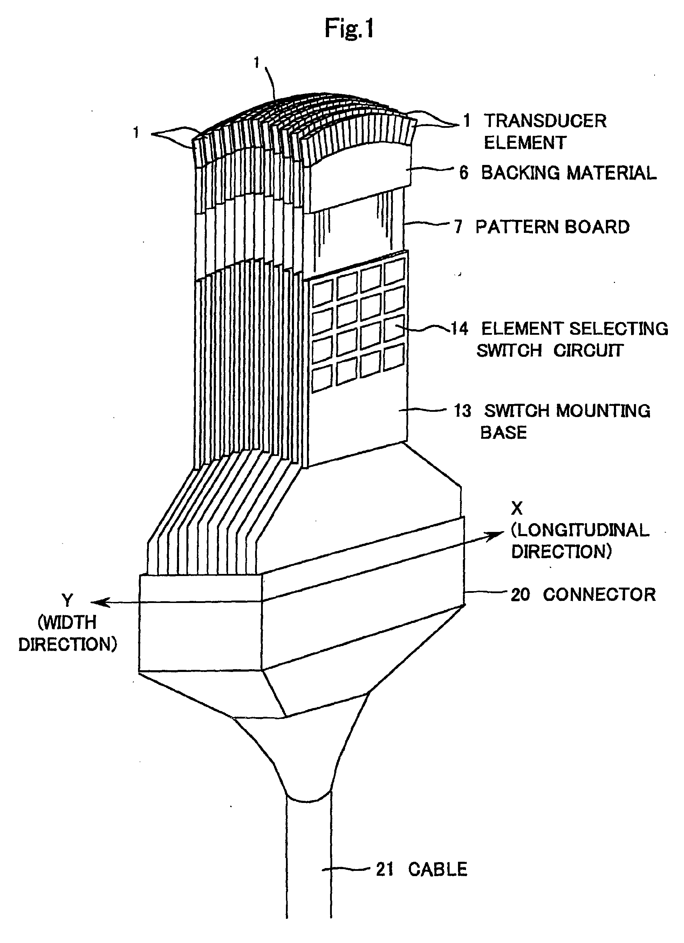

Fig.1 is a perspective view showing an embodiment of an ultrasonic probe of the present invention. The ultrasonic probe comprises a plurality of transducer elements arranged two-dimensionally for transmitting and receiving ultrasound to an object to be examined. In the ultrasonic probe, the plurality of transducer elements 1, for example 12,488 elements, are arranged in longitudinal direction (X direction) and in transversal direction (Y direction) with 196 × 64 elements, or 6,244 elements are arranged to longitudinal direction (X direction) and transversal direction (Y direction) with 128 × 48 elements. They are arrayed with a two-dimensional convex shape in the ultrasound transmitting direction with one direction being the longitudinal direction and the other the direction across.

The circular angle formed with the transducer elements in the longitudinal direction x, is about 68 degree for example 196 or 128 elements, and the circular radius forming the ultrasound transmitting and receiving face is about 60 mm. The circular angle formed with the transducer elements in the transversal direction Y, is about 48 degrees for example with 64 or 48 elements and the circular radius of the ultrasound transmitting and receiving face is about 32 mm. A three-dimensional scanning with ultrasonic beams is possible within the field of view of 68 degrees × 48 degrees.

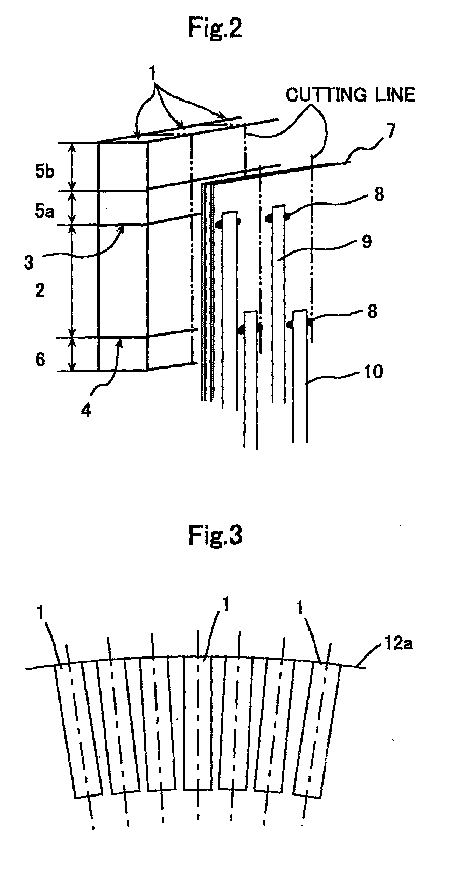

The transducer elements 1 transmit an ultrasound to an object and receive a reflection wave from the object. They consist of a piezoelectric material for changing mutually between an electric signal and a mechanical vibration. The structure of one line of transducer elements 1 in the longitudinal direction X is shown in Fig.2. A ground electrode 3 is fixed on the top of the piezoelectric material 2, and a signal electrode 4 is fixed on the bottom of it. Furthermore, two matching layers (acoustic adjustment layer) 5a, 5b are fixed on top of the ground electrode 3, and a sound attenuation material (backing material) 6 is fixed on the bottom of the signal electrode 4.

The piezoelectric material 2, in which the matching layer 5a, 5b and the backing material 6 is fixed on the top or the bottom of it, is previously cut to a thin plate. A pattern board 7 is fixed with solder to the ground electrode 3 and the signal electrode 4 at the exposed sides of it. On the pattern board 7, a ground electrode pattern 9 and a signal electrode pattern 10 are formed, which are connected to the ground electrode 3 and the signal electrode 4 through a solder pad 8. In the case that the pattern board 7 is fixed as described, each transducer element 1 is formed by cutting the whole piezoelectric material 2 separately with a dicing saw along a cutting line 11 shown as a chain double-dashed line. In addition, it is preferable to cut the whole piezoelectric material 2 separately along the cutting line 11 before the pattern board 7 is fixed by the solder and to arrange the transducer elements 1 respectively in accordance with a ground electrode pattern 9 and a signal electrode pattern 10 of the pattern board 7.

The arrangement of the plurality of transducer element 1 arrayed in the longitudinal direction X is shown in Fig.3. For example, 192 transducer elements 1 are arrayed in the longitudinal direction X. The top face of them is positioned along a longitudinal arrangement orbit 12a. The transducer elements 1 are arrayed as a whole with circular convex and radial arrangement in the transmitting direction, which is called convex type. In this embodiment, the center axis of each element 1 is radiated outward Y. The ultrasonic transmitting and receiving face of each element 1 is arranged to the normal direction of the longitudinal arrangement orbit 12a. Accordingly, the high sensitivity direction for transmitting and receiving ultrasound of each transducer element 1 is faced to the normal direction of the longitudinal arrangement orbit 12a. Therefore, especially a signal receiving sensitivity at both ends of the transducer array is improved.

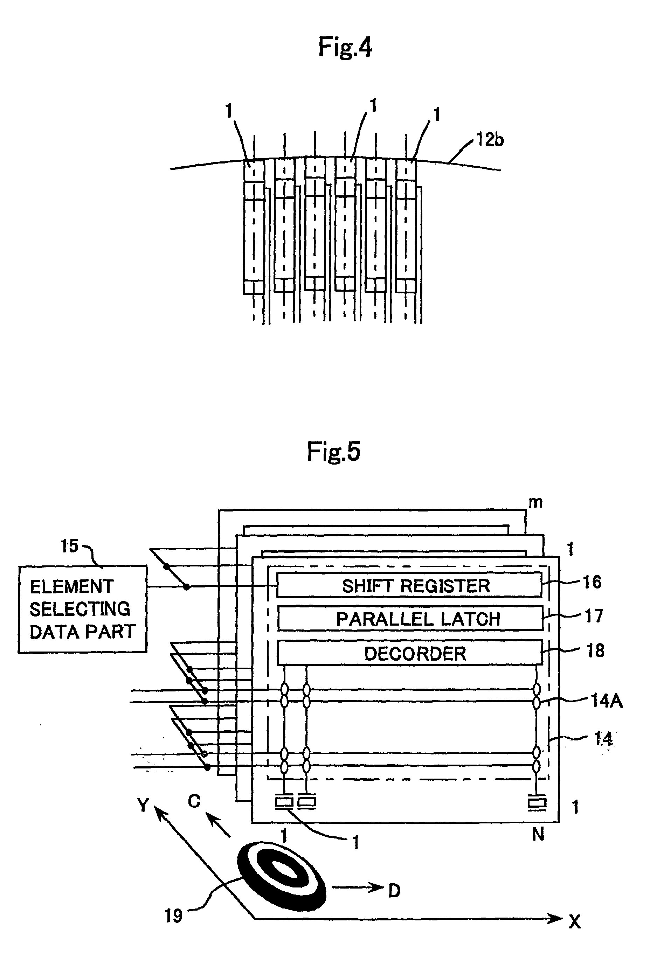

In addition, an arrangement of the plurality of transducer elements 1 arrayed in the transversal direction Y of Fig.1 is shown in Fig.4. For example, 64 transducer elements 1 are arrayed in the transversal direction Y. The top face of them is positioned like a stair along a transversal arrangement orbit 12b. They are arrayed with a circular convex arrangement in the transmitting direction so called convex type. In this embodiment, the center axis of each element 1 is arranged parallel. Both ends of the transducer array 1 are arranged to be inclined with a certain degree to the normal direction of the transducer arrangement orbit 12b. Therefore, the signal receiving sensitivity falls down a little at both ends of the transducer array.

As previously described, a two-dimensional probe of the present invention is applied with the convex shape in both directions - longitudinal and transversal. Accordingly, the fitness of the probe to a surface of the object is improved.

In Fig. 1, each transducer element is connected to an element selecting switch circuit 14 on a switch mounting base 13 through the pattern board 7. This element selecting switch circuit 14 selects an arbitrary transducer element for transmitting and receiving ultrasound. They are connected respectively to each of said plural transducer elements on said two-dimensional array. In Fig. 5, n pieces of transducer elements are arranged in the longitudinal direction X, and m pieces of transducer element in the transversal direction Y. All of them are connected to an element selecting switch 14a, for example composed of a cross point switch. In two-dimensional probes composed like these, the transducer elements for actually transmitting and receiving ultrasound are selected arbitrarily by control of the element selecting switch 14a through a shift register 16, a parallel latch 17 and a decoder 18 in the transducer array with a selecting data input from an exterior element selecting data part 15.

In the following, an example is given for forming an arbitrary shape diameter for transmitting and receiving ultrasound by selecting an arbitrary transducer element with the control of said element selecting switch circuit 14. As shown at the bottom of Fig.5, a plurality of transducer elements is selected from the two-dimensional array of n pieces in the longitudinal direction (x direction) and m pieces in the transversal direction (y direction) by control of the element selecting switch circuit 14. Those elements are selected which form for example a concentric circle with diameter 19. A fresnel ring is composed of the concentric circles formed by connecting and bundling of the transducer elements in the concentric ring. It is possible to form a focusing beam by the fresnel ring with a divided annular array. In addition it is possible to form equally a divided annular array and other types of arbitrary shape array. The fresnel ring, in which the transducer elements are bundled with concentric circle, is disclosed in USP 4,219,846.

Furthermore, the two-dimensional probe of the present invention can transmit and receive an ultrasonic beam with the continuously moving diameter 19, which composes of the fresnel ring, by controlling the element selecting switch circuits 14 at each repetition of the ultrasound transmitted and received to the two directions shown in Fig.5 as arrows C and D on the face of the probe. As the ultrasonic probe can scan with the ultrasound beam in a third direction in a certain range, the probe can get three-dimensional image data. In addition, the shape of the diameter 19 is not restricted to the fresnel ring shape, in which the transducer elements are bundled into concentric circles, but it is possible to bundle them into an oval, a rectangular, or another arbitrary shape by changing the transducer selecting switch circuit 14. In this case, the ultrasonic beam focused with an arbitrary shape is transmitted and received from the diameter 19.

Reference is made to Fig. 1 again. Reference 21 shows a cable for connecting the two-dimensional probe to the main body of an ultrasonic diagnostic apparatus (not shown). Reference 20 shows a connector for connecting the transducer elements of the two-dimensional probe to the cable 21. As the element selecting switch circuit 14 is controlled by a device disposed in the main body through the cable 21 and the connector 20, the arbitrary transducer element is selected to form an arbitrary diameter for transmitting and receiving ultrasound. A focused ultrasound beam with arbitrary shape can be transmitted and received from the diameter to scan three-dimensionally. Accordingly, in the ultrasonic probe of the present embodiment cable 21 is needed only for at least the number of transducer elements forming an arbitrary shape diameter for transmitting and receiving ultrasound and for the number of signal lines for supplying transducer selecting data. Therefore, the probe is smaller and lighter.

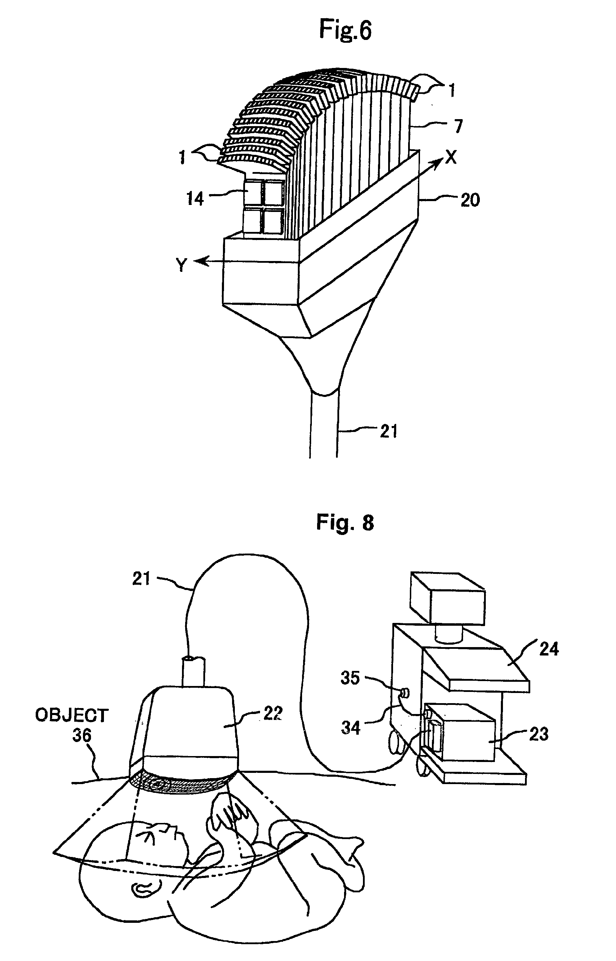

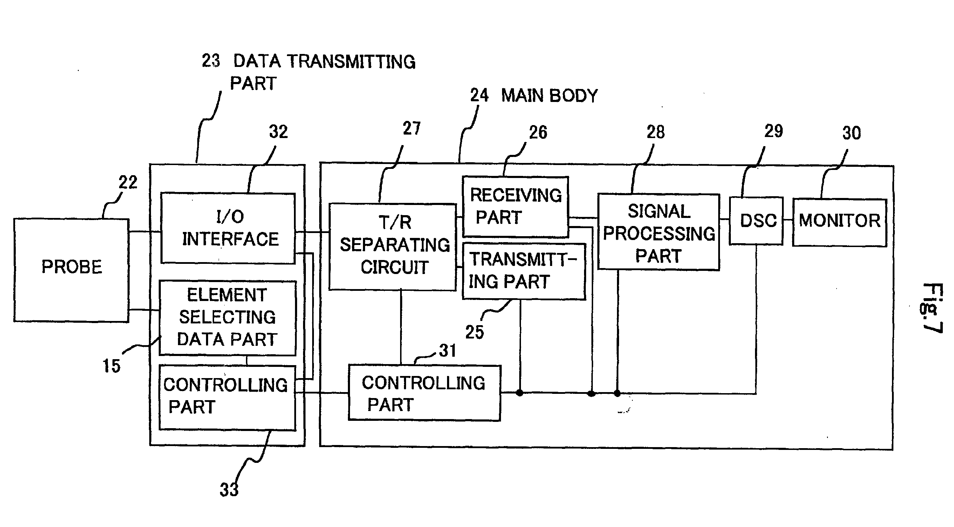

Fig.6 is a perspective explanation view showing a modified example of the ultrasonic probe shown in Fig.1, and a schematic view showing the interior structure of it. In this modified example the plurality of transducer elements 1 are arranged with a convex type, which has a circular convex shape in the longitudinal direction X as shown in Fig.3 and a radial arrangement in the ultrasound transmitting direction. They are convexly arranged in the transversal direction Y as shown in Fig.3.

In this embodiment, the high sensitivity direction of the transmitted and received ultrasound of each transducer element 1 is turned to the normal direction of the longitudinal arrangement orbit 12a in the longitudinal direction X. A high sensitivity direction of the transmitted and received ultrasound of each transducer element 1 in the transversal direction Y is turned to the normal direction of the transversal arrangement orbit 12b. The arrangement is ideal to scan in the longitudinal direction X and the transversal direction Y. Especially the receiving sensitivity on both ends of the transducer array are improved.

The plurality of transducer elements is arranged with a circular convex shape in each of the two directions being perpendicular to each other in the ultrasound transmitting direction. Accordingly, the two-dimensional probe has good contact with the surface of the object to be examined. The fitness of the probe to the object can be improved by arrangement with a convex shape in at least one direction of the two-dimensional array.

An element selecting switch circuit for selecting an arbitrary transducer element for transmitting and receiving ultrasound is disposed in the vicinity of the two-dimensional array transducer elements. An arbitrary transducer element can be selected by controlling on and off of the element selecting switch circuit, and the plurality of transducer elements can be connected in common (bundled connection). Therefore, the number of lines for connecting the main body and the probe can be reduced. Accordingly, an operator's load during scanning can be reduced.

Fig.7 is a block diagram for showing a further embodiment of the ultrasonic diagnostic apparatus of the present invention. The ultrasonic diagnostic apparatus gets and displays an ultrasonic image of a diagnosis part in the interior of the object by using ultrasonic signals corrected with transmitting and receiving ultrasound to the object. As shown in Fig.7, it comprises a probe 22, a data transmitting part 23, and a main body 24.

The probe 22 transmits and receives ultrasound to the object to be examined. In the present embodiment, the ultrasonic probe shown in Fig.1 or Fig.6 or the ultrasonic probe with the two-dimensional array transducer on a plane is used.

In addition, the main body 24 of the diagnostic apparatus gets and displays an ultrasonic image of the diagnosis part in the interior of the object by using ultrasound signals corrected by the probe 22. The apparatus is composed as a traditional ultrasonic diagnostic apparatus used in general. The main body 24 comprises a transmitting part 25 for supplying a transmitting signal to the transducer elements with a delay time controlled by a transmitting delay circuit or a timing control such that a transmitted ultrasound from said probe to the object is focused at a desired focused point. The main body 24 further comprises a receiving part 26 for forming a receiving beam signal by a beam forming process about an echo signal received with said probe 22, a transmitting and receiving separation circuit 27 for exchanging the connection of said probe 22 to the transmitting part 25 or the receiving part 26 corresponding to the transmitting or receiving of ultrasound, a signal processing part 28 for performing a detection processing, a logarithm compression processing, an edge emphasis processing or the like by inputting a received signal from the receiving part 26, a digital scan converter (DSC) 29 for forming an image data from input data from the signal processing part 28, together performing a scan transformation and interpolation processing or the like for displaying an image, a monitor 30 for displaying data inputted from the DSC 29 as an ultrasonic image, and a controlling part 31 including CPU for controlling operation of each units.

Furthermore, in the present embodiment, a data transmitting part 23 is disposed between the probe 22 and the main body 24. The data transmitting part 23 is connected between main body 24 and probe 22 for inputting and outputting selecting data of transducer elements and the corrected ultrasound signal. It comprises an element selecting data part 15, in which an element selecting data is accommodated to form a diameter (hereinafter described) for transmitting and receiving ultrasound by controlling the element selecting switch 14 (refer to Fig.5) disposed in the vicinity of the transducer of the ultrasonic probe shown in Fig.1 and Fig.6, and an input and output interface 32 for performing the transmitting and receiving of ultrasound by connection of probe 22 and main body 24, and a controlling part 33 for controlling the element selecting data part 15 and the input and output interface 32 to enable transmitting and receiving ultrasound with the selected transducer elements.

The controlling part 33 is also connected to controlling part 31 in the main body 24. The element selecting data part 15 is comprised of, for example a ROM (Read only memory), to memorize an element selecting data for forming a diameter of transmitted and received ultrasound. The data readout from the element selecting data part 15 is transferred to a shift register 16 as shown in Fig.5. The on and off switching of the element selecting switch circuit 14 is controlled to form a diameter of transmitted and received ultrasound through the shift register 16, the parallel latch 17 and the decoder 18.

Fig.8 is an outline diagram showing an operating state of an ultrasonic diagnostic apparatus. The cable 21 stretched from the ultrasonic probe shown in Fig.1 or Fig.6 is connected to a data transmitting part 23, and a cable 34 stretched from the data transmitting part 23 is connected to a connector 35 of the main body 24. The probe 22 is contacted to an object 36 to be examined. As described above, by arranging the data transmitting part 23 between the probe 22 and the main body 24, it is possible to connect the two-dimensional array probe to a traditional and general ultrasonic diagnostic apparatus without using an ultrasound diagnostic apparatus for the exclusive use of the two-dimensional array probe.

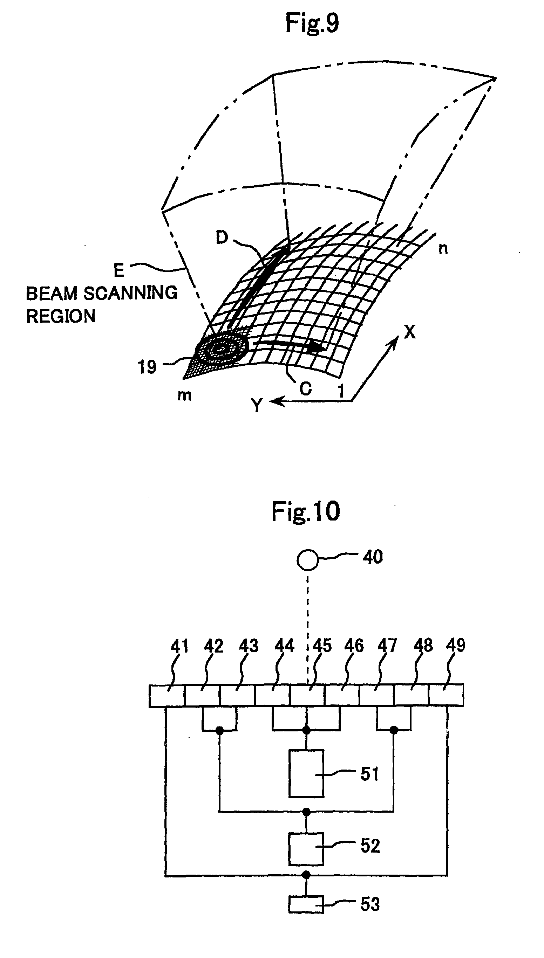

Next, an embodiment for transmitting and receiving ultrasound in said ultrasound diagnostic apparatus will be described. Fig.9 shows a concept for a three-dimensional scanning of the object to be examined. In Fig.9, the two-dimensional array transducer 1 has the sum of m × n transducer elements, n in X direction and m in Y direction, with X and Y being perpendicular, which results in 12,488 or 6,244 pieces for example. The transducer groups (diameter) 19 driven for transmitting and receiving in its array are formed. The diameter 19 is composed of a plurality of concentric rings (fresnel rings). So a fresnel ring, for example, with 32 concentric rings in which each ring has 64 transducer elements, or with 16 concentric rings in which each ring has 58 transducer elements or the like can be raised. But the number of rings and elements forming one ring can be determined in accordance with the system composition. To form each ring with the same number of elements or with other reason, the width of the ring is narrowed as it goes to the exterior. The reason will be described later.

The diameter 19 is moved in the X or Y direction at each cycle of ultrasound transmitting and receiving, or in the C or D direction along the arrangement face of the transducer with a predetermined order. At each position of the diameter during the moving process, an ultrasound is transmitted from the diameter 19 to the object to be examined, and the reflection wave is received. As a result, the three-dimensional scanning is performed to the interior of the object with an ultrasonic beam.

Fig.10 shows the connection between the transducer elements for transmitting and receiving and the delay circuits in the two-dimensional array probe of the present embodiment. In Fig. 10, references 41 to 49 refer to transducer elements, references 51 to 53 to delay circuits, and reference 40 is a the convergence point of ultrasound (focus point). The transducer elements 44, 45, 46 form the first ring, and the transducer elements 42, 43 and 47, 48 positioned at the exterior of them form the second ring, which radius is larger than the former. Transducer elements 41, 49 are positioned at the further exterior form the third ring, which has the largest diameter. As described above, the ring width of the outer ring is narrower than that of inner ring. In an actual device, the number of rings is 32 or 16, and the elements are divided with the idea as shown in Fig.10.

The transducer elements 44, 45, 46 forming the first ring are connected commonly (bundling connection) to a delay circuit 51. The transducer elements 42, 43, 47, 48 forming the second ring are connected to a delay circuit 52 with bundling, and the transducer elements 41, 49 forming the third ring are connected to a delay circuit 53 with bundling. The reason for using the bundling connection method is to reduce the number of delay circuits, and to provide a compact device. In case the bundling connection is not used, a delay circuit and a delay data are needed for each element composing the fresnel ring. If fresnel ring is formed with 2,048 or 928 elements, the same number of delay circuit and delay data is needed and the device becomes very large and the costs raise.

Although one delay circuit is composed at each ring in Fig.10, the main purpose shown in Fig. 10 is that the transducer elements forming one ring are controlled with the same delay time for transmitting the ultrasound and receiving the signal. Accordingly, it is preferable to arrange a delay circuit for applying the same delay time to the signal at every region formed by dividing one ring to a plurality of regions. But in this case, it is apparent that the number of delay circuit is less than the number of elements for forming the ring. In addition, it is preferable to change the number of rings to the number of delay circuit; a connecting state is shown in Fig.10 with transmitting and receiving time. The acoustic field of ultrasound transmitted from the transducer of the fresnel ring has a narrow characteristics, so it is preferable that the delay control is performed to a roughly divided ring in transmitting, and to a finely divided ring in receiving.

The ring forming and the connecting of delay circuits is controlled with the controlling part 31 and the controlling part 33. The controlling part 31 controls transmitting and receiving of the ultrasound and the image forming and displaying in the whole ultrasonic diagnostic apparatus. The controlling part 33 receives an ultrasound transmitting and receiving command and a scanning command from the controlling part 31, and controls the selection of transducer elements serving for the transmitting and receiving of ultrasound, and the connection of transducer elements and delay circuits. The controlling part 33 receives the commands from the controlling part 31 at each cycle of transmitting and receiving, and sends a transducer element selecting command to the element selecting data part 15 for performing the next transmitting and receiving. Corresponding to the transducer element selecting command, the signal for forming the fresnel ring and the signal for performing the connection change of the fresnel ring and the delay circuit are output from the element selecting data part 15 to the element selecting switch circuit 14.

The selecting switch circuit 14 has m×n×s switching elements in case that the two-dimensional probe 22 has m×n elements and the selecting switch circuit has s input and output lines. If the number of transducer forming a whole fresnel ring is F, and each ring has the same number of transducer, and the number of rings is N, then the number of input and output lines to the selecting switch circuits 14 is also N. One of the selecting switch circuits N is connected to one ring and one ring has F / N transducer elements. In addition, a connecting end of input and output lines from the selection switch circuit 14 is transmitting the delay circuit in the transmitting part 26, or the beam forming circuit in the receiving part 25. Its number is the same as the number of transmitting and receiving channels being able to control independently in the main body. Generally, it is a multiplier of 2 and more than 16.

The transmitting delay circuits sets the ultrasound focus depth in the object to be examined when the ultrasound is transmitted. The beam forming circuit moves dynamically the focusing depth at the signal receiving (this is called dynamic focusing) and adds signals output from each channel with matching their phase. For example, a delay time data applied to the transmitting delay circuit is set with the apparatus corresponding to a predetermined depth in the region of interest (ROI) of the object input by an operator. The delay time data for moving a focus point is given automatically with the apparatus to the overall depth for the measuring signal to the delay circuit in the beam forming circuit.

The delay time data are calculated by calculations based on the distance value determined with geometrical arrangements of the transducer elements and the focus points, or are previously accumulated and accommodated in the ROM of the controlling part 31.

Hereinafter the operation of the ultrasonic diagnostic apparatus shown in Fig.7 will be described. An operator brings the probe 22 in contact with the examined part of the object and inputs an ultrasound scanning command to main body 24. Then the controlling part 31 sends an ultrasound scanning beginning command to each unit. The controlling part 33 receives the command and outputs an element selecting command to the element selecting data part 15 for performing the first ultrasound transmitting and receiving. Corresponding to this commands, an on / off command for each switch in the element selecting switch circuit 14 is output from the element selecting data part 15. The transducer elements serving for the first transmitting and receiving in the probe 22 are selected with an on / off operation of each switch at the element selecting switch circuit 14. The selected transducer elements form the fresnel ring. Transducer elements forming each ring of the fresnel ring are connected by bundling. When this connection is completed, the transmitting pulse signals are output from the transmitting part 25. The transmitting pulse provided delay time is applied to each fresnel ring. The transmitting pulse is guided to an input and output line of the interface 32 by transmitting and receiving separation circuits 27 and is provided to each ring of the two-dimensional probe 22. Thus, a focused ultrasound is irradiated to the predetermined depth in the object from probe 22.

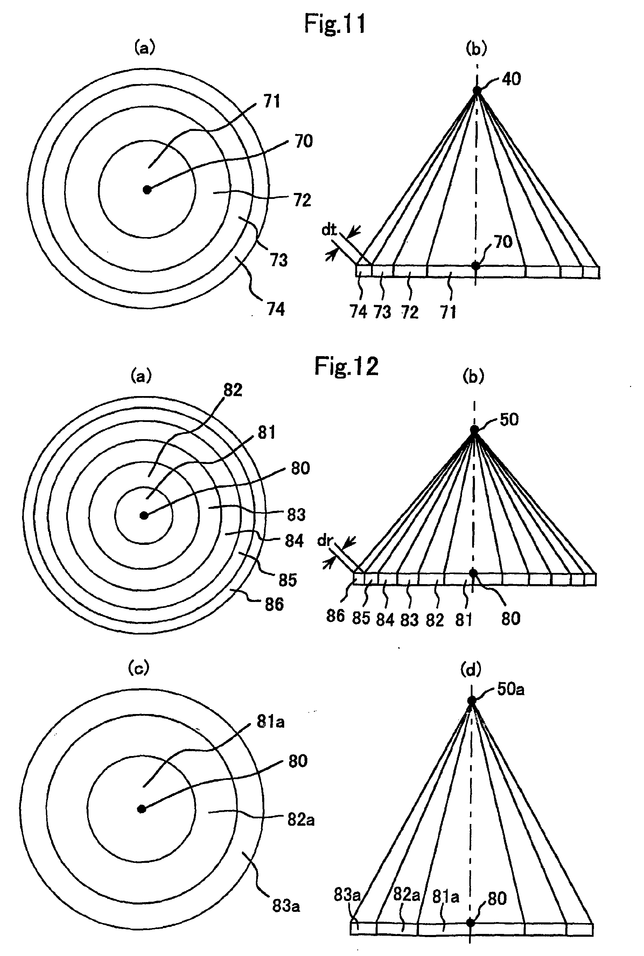

The forming method of the fresnel ring when a transmitting wave is focused will be described with reference to Fig.11. In Fig.11, reference number 70 refers to a center of the fresnel ring, 71, 72, 73, 74 are fresnel rings formed by the plurality of transducer elements. The exterior circle of the fresnel ring 74 has a diameter 19. In the following, the relationship between the radius of the ring and the focus point is explained. In all fresnel rings, in case the transmitting focus point 40 is located deeper than 50 mm in the object, it is preferable that the difference dt between a maximum and a minimum value of the distance between the transmitting focus point 40 and the transducer elements in each ring, is less than 1 / 2 wave length of the ultrasound emitted from the probe, or more preferably less than 1 / 8 wave length of the ultrasound of the probe. The reason is that, for example, when dt is 1 / 2 wavelength, the phase of the associated ultrasound differs of about 180 degrees. Then the ultrasonic wave is canceled with its associated wave. As the best region being able to allow the influence of this canceling, the distance is selected less than 1 / 8 wavelength. In addition, when the depth of the transmitting focus point 40 is needed to be set less than 50 mm, for example in case of the examination of a carotid artery, the value of dt may be more than 1/8 without changing the diameter. In this case, it is preferable to reduce the diameter by changing the width of each ring without changing the number of rings of the fresnel ring for transmitting. Alternatively, it is preferable to reduce the diameter substantially by driving only rings satisfying the condition in the center part. Moreover, it is preferable that the signal is received with the small diameter until the depth that value of dt is more than 1/8 wavelength is reached, and when it is deeper than that, the signal is received with the larger diameter. In the present embodiment, a consistent delay time controlling is performed for each ring, so the ultrasonic beam is formed in the perpendicular direction to the ultrasound transmitting surface. From the setting method of the value dt, the fresnel ring 71 is a concentric circle with the center of diameter. And the width of the exterior concentric ring becomes narrower.

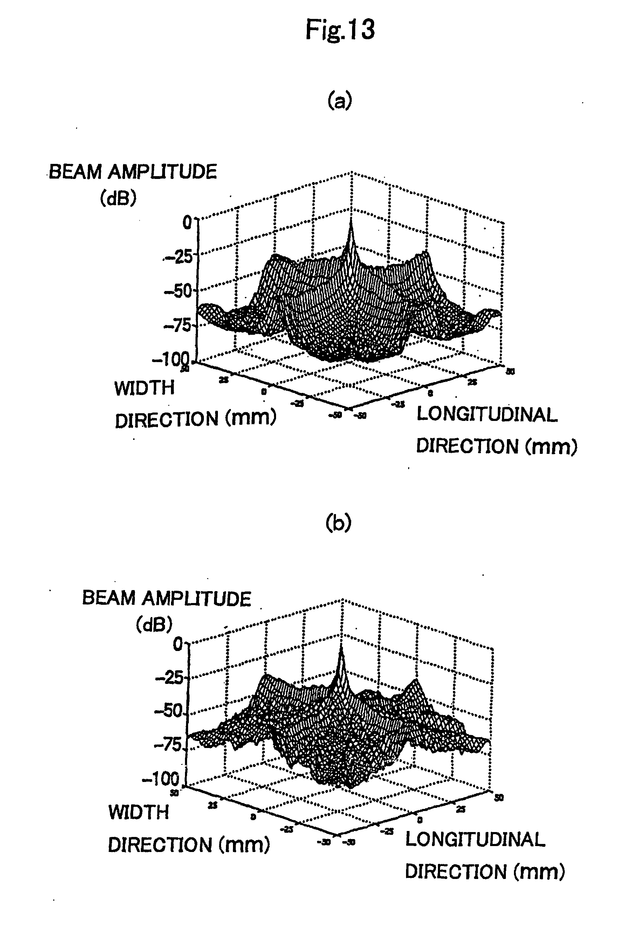

Next the validity that the difference between the maximum value and the minimum value of the distance between the transducer elements and the focus point in each ring, is made to be less than 1 / 8 wave length will be explained with reference to Fig.13. Fig.13 shows the beam amplitude (sound pressure) of a two-dimensional ultrasound beam calculated by simulation at a depth of 50mm with the focus point being located 50mm in front of diameter by using the two-dimensional convex type probe with an ultrasound transmitting and receiving face convexly at both directions, in which an ultrasound frequency is 3.5 MHz, the curvature radius in the longitudinal direction is 40 mm, the curvature radius in the transversal direction is 25 mm, and an element pitch is 0.38×0.38mm. In addition, the diameter used for this simulation has 64 elements in the longitudinal direction, and 32 elements in the transversal direction.

Fig.13 (a) shows an ideal beam in case the delay controlling is performed to all 2,048 elements of the diameter. Fig.13 (b) is an amplitude distribution figure of a beam when the diameter is divided to 32 fresnel rings and the delay controlling is performed with fresnel bundling. The latter fresnel bundling is performed such that the difference between the maximum and the minimum of the distance between the elements of the ring and the focus point, is made to be less than 1/8 wavelength. Comparing the two figures, the main beam width of the latter figure is the same as the ideal beam in Fig. 13a, and the useless response is less than minus 40dB. Accordingly, it will be no problem in practical use.

Next, the receiving operation will be described. When the ultrasound is transmitted from the ultrasonic probe 22 to the object, the controlling part 31 sends a command for the receiving operation. The ultrasound transmitted from the ultrasonic probe 22 to the object reflects at the boundary where the acoustic impedance in the object is different. The reflecting signal is received with the same two-dimensional probe 22. At this time, the element selecting data part 15 holds the data that is used for transmitting. Accordingly, the fresnel ring and the signal transmitting are formed in probe 22. In addition, when the signal is received, the form of the fresnel ring (ring number, number of elements for forming one ring and diameter) can be changed.

In the following, an example to modify the form of the fresnel ring in comparison with transmitting will be described. Although in the present invention the dynamic focusing is performed when a signal is receiving, it is preferable to form the fresnel ring at the dynamic focusing as shown in Fig.12. In Fig. 12 (a), reference 80 is a center of fresnel ring at receiving, 81, 82, 83, 84, 85, 86 are fresnel rings, 50 is a receiving focus point. The exterior circle of the fresnel ring 86 is the receiving diameter. And as shown in Fig.12 (b), it is preferable that the difference dr between a maximum value and a minimum value of the distance between the receiving focus point 50 and the elements in one ring, is less than a certain value as in the transmitting case, preferably less than 1 / 8 wave length of the ultrasound.

In the present invention, the same delay time controlling is performed at each ring in receiving as in transmitting, so the ultrasonic beam is formed on a perpendicular line that passes through the center of the fresnel ring 80. Accordingly, when the focus point 50 changes from short distance to long distance, the fresnel ring is maintained in concentric circles within the center of the ultrasound receiving diameter. When the focus point is closer to the ultrasound receiving face, the distance between the element and the focus point is larger. As the method for dealing with this, in case the focus point 50 is close to the face, the fresnel ring can be composed with a concentric ring having relatively narrow width as shown in Fig.12 (a), (b). In case the focus point is located at 50a with long distance, the fresnel ring can be composed with a concentric ring having a small number of rings and a relatively wide width 81a, 82a, 83a as shown in Fig.12 (c), (d). Moreover, it is preferable to receive only with the central part when the focus point 50 is at short distance, and to increase the number of rings in accordance with the focus point 50 becomes longer distance. Therefore, it is preferable to apply a variable diameter method. Furthermore, it is preferable to modify the shape of the fresnel rings between the short distance region and the long distance region of focus points, and to divide each region to scan. It is preferable to combine each separately acquired echo signals for imaging.

Furthermore, in another embodiment, it is preferable to fix the fresnel ring with a short distance pattern as shown in Fig.12 (a), (b) during receiving dynamic focusing. Fixing the fresnel ring with the short distance pattern, when it is used for the long distance, the difference between the maximum and the minimum distance between the focus point and the elements in each fresnel ring, becomes small in comparison with the short distance case. For example in certain fresnel rings, if the difference dr between the maximum and the minimum value between the focus point and the element at a short distance is 1 / 8 wave length, at a long distance the difference dr will be for example 1 / 16 wave length of the ultrasound. Accordingly, an accuracy of the delay to the signal is improved in comparison with the case of the short range. Therefore, the ultrasonic beam does not deteriorate at long distance focusing.

In addition, in case the channel number of the beam forming circuit disposed in the receiving part 26 is small, if the fresnel ring is fixed in the pattern for short range, sometimes the width of the fresnel ring becomes narrow and the diameter is small. In this case, it is necessary to define a fitting pattern of fresnel ring with the consideration of both the difference dr between the maximum and the minimum of the distance between the focus point and the element in each fresnel ring and the diameter.

Hereinafter, moreover the receiving operation will be described. For starting the receiving operation, the controlling part 31 supplies successively the focusing data to all beam forming circuits to perform the dynamic focusing in the receiving part 26 to continuously move the receiving focus point from the shallow part to the deep part of the object to be examined. Thus, the echo groups received with the fresnel ring are aligned in their phase with the beam forming circuit, and are output as ultrasonic beam signal (echo signal) with an addition circuit. The signal processing part 28 performs the detection processing, logarithm compression, filtering processing, edge emphasis processing or the like to output the signal and sends processed signals to the DSC 29. The DSC 29 converts the input signals to digital signals, thereafter writes them to a memory build in it. By the above-mentioned operation, the first ultrasound transmitting and receiving operation is completed.

Next, the controlling part 31 sends the second ultrasound transmitting and receiving command to each unit. Although the first transmitting and receiving is performed at a position shown in Fig.9, the second transmitting and receiving is performed by forming the fresnel ring at a position moved by a predetermined distance to a certain X or Y direction. The next received echo signal is written in the memory in DSC 29 as same as the first transmitting and receiving. After this, the position of fresnel ring on the probe is moved similarly to the predetermined direction consecutively, and the two-dimensional scanning is performed in the object with the ultrasonic beam. When the two-dimensional scanning is completed, one slice image of the object is formed in the memory of DSC 29. The controlling part 33 reads out the slice image data in the memory of the DSC 29 in synchronization with a horizontal scanning for displaying on a monitor. The slice image is displayed on the display screen of monitor 30. After the two-dimensional scanning is completed, the controlling part 31 performs the two-dimensional scanning again with moving the fresnel ring on the probe from the position in the two-dimensional scanning to a different position in the scanning. With this repetition, beam scanning region E in the object to be examined as shown in Fig.9 is scanned three-dimensionally with the ultrasonic beam. With this three-dimensional scanning, a plurality of slice data is acquired to accommodate to the memory in DSC 29. Those slice image data are a three-dimensionally image processed with a three dimensional image reconstruction method such as surface rendering method or volume rendering method etc. by the controlling part 33 to be displayed at a screen of the monitor 30 as a three dimensional image. A diagnosis in which this ultrasonic three-dimensional image is used includes for example a deformity diagnosis of an unborn baby before birth at an obstetrics and gynecology department or the like. It will be understood that a modified example is possible, and it is preferable to perform image processing for forming a three-dimensional image with image processing device composed separately from the main body.

Furthermore, in a setting method for the diameter shown in Fig.9, all fresnel rings moved are set to draw a perfect circle at the end of ultrasonic probe. But in this method, the ultrasonic beam in the most outer edge is interior of the outline of the transducer arrangement face of the probe with the radius of the fresnel ring. So, the scanning region of the ultrasonic beam is small. To solve this problem, when the ultrasonic beam at the most outer edge is transmitted and received in the three-dimensional scanning with the present probe, it is preferable to form an imperfect fresnel ring, for example a fresnel ring of 1 / 2 circle and to locate the ultrasonic beam to the edge of the transducer array.

According to the second embodiment of the present invention using a data transmitting part between the probe and the main body, the three-dimensional scanning can be performed with an ultrasonic beam to the object with connecting the ultrasonic probe of the first embodiment to the traditional and general ultrasonic diagnostic apparatus without using an ultrasonic diagnostic apparatus for only two -dimensional array probe.

In addition, according to the ultrasonic diagnosis device of the present invention, the ultrasonic beam is usually perpendicular to the ultrasonic transmitting and receiving face and formed in the center of the fresnel ring. So in case the location of the focus point is changed from short distance to long distance, the form of the fresnel ring need not be changed. This is a characteristic of the present invention. The element bundling in the processing of received signal is not needed. So both - fresnel bundling and receiving dynamic focusing - can be performed. Moreover, the receiving dynamic focus can easily be performed.

## Claims

1. An ultrasonic probe having a plurality of transducer elements arranged two-dimensionally for transmitting and receiving ultrasound, wherein said plurality of transducer elements is arranged with convex shape in two directions being perpendicular to each other relative to the ultrasound transmitting direction.

2. The ultrasonic probe of claim 1, wherein an element selecting switch circuit for selecting an arbitrary transducer element is disposed in the vicinity of said two-dimensionally arranged transducer elements for performing the transmitting and receiving of ultrasound.

3. The ultrasonic probe of claim 2, wherein said element selecting switch circuit has output lines for connecting each transducer element, input lines wherein the number of input lines is less than the total number of arranged transducer elements, and a control line for inputting a control signal for performing a transducer selecting change.

4. The ultrasonic probe of claim 2, wherein a control signal given to said element selecting switch circuit selects an arbitrary element from a transducer elements array to form a diameter of transmitted and received ultrasound, and this diameter is movable to an arbitrary position at each ultrasound transmitting and receiving cycle.

5. The ultrasonic probe of claim 4, wherein said diameter is moved in two directions of the two-dimensional array of transducer elements in a predetermined order.

6. The ultrasonic probe of claim 1, wherein the two-dimensionally arranged transducer elements are arrayed radially in one and the other direction of the arrangement.

7. The ultrasonic probe of claim 6, wherein an ultrasonic transmitting and receiving face of the transducer elements is formed convexly and radially with a radius of 60mm and a circular angle of about 68 degrees in one direction of the arrangement, and in the other direction with a radius of 32 mm and a circular angle of about 48 degrees.

8. The ultrasonic probe of claim 1, wherein the two-dimensionally arrayed transducer elements are arrayed radially in one direction, and parallel along the center axis of a circle in the other direction.

9. The ultrasonic probe of claim 8, wherein an ultrasound transmitting and receiving face of the transducer elements is arrayed convexly and radially with a diameter of 60mm and a circular angle of about 68 degrees.

10. An ultrasonic probe having a plurality of transducer elements for transmitting and receiving ultrasound in two directions, wherein said plurality of transducer elements is arrayed convexly to the ultrasound transmitting direction at least in one direction of the two dimensional array or another direction perpendicular to said direction of the two dimensional direction.

11. The ultrasonic probe of claim 10, wherein a switch circuit for selecting a transducer element from said two-dimensional arrayed transducer elements is disposed to form an ultrasound transmitting and receiving diameter which is smaller than the length of both sides of the two-dimensional arrangement.

12. An ultrasonic diagnostic apparatus comprising;
an ultrasonic probe with two-dimensionally arrayed minute ultrasonic transducer elements,
an element selecting means for selecting transducer elements to perform the transmitting and receiving of ultrasound from a transducer of said elements array ultrasonic probe,
means for supplying bundling data to said element selecting means for bundling and connecting transducer elements to a plurality of groups,
means for transmitting ultrasound to an object with a predetermined transmitting delay time for each bundling connected transducer element group,
means for beam forming each receiving signal output from said bundling connected transducer element group,
means for image processing an output signal of the beam forming means, and an image display means.

13. The ultrasonic diagnostic apparatus of claim 12, wherein the plurality of bundling connected transducer element groups form a fresnel ring of concentric circle.

14. The ultrasonic diagnostic apparatus of claim 13, wherein the difference of a maximum and a minimum of the distance between the transducer elements forming a fresnel ring in each ring and an ultrasonic focus point is less than 1 / 8 wave length of ultrasound.

15. The ultrasonic diagnostic apparatus of claim 13, wherein the means for controlling said beam forming circuit is composed to receive a signal of each ring such that the receiving focus point is moved continuously on the center line of the fresnel rings without changing the form of said fresnel ring in the receiving period of an echo signal.

16. The ultrasonic diagnostic apparatus of claim 13, wherein a means for changing the form of said fresnel ring corresponding to the depth of receiving focus point is provided.

17. The ultrasonic diagnostic apparatus of claim 16, wherein a means for scanning the predetermined region of depth within the object at each form of said fresnel ring, and for composing an image from an echo signal acquired at each region of depth is provided.

18. An ultrasonic diagnostic apparatus comprising;
a two-dimensional array ultrasonic probe for transmitting and receiving ultrasound to an object,
a main body of a diagnostic apparatus for acquiring and displaying an ultrasonic image of a diagnosis part in the interior of the object by using an ultrasonic signal corrected with said two-dimensional array probe,
a data transmitting part for transmitting a selecting data of transducer elements and the corrected ultrasonic signal between said two-dimensional array ultrasonic probe and said main body, and for connecting the probe and the main body.
